# EUROPEAN PATENT SPECIFICATION

(11) **EP 0 472 725 B1**
(45) Date of publication and mention of the grant of the patent: **21.05.1997**
(21) Application number: 91909886.3
(22) Date of filing: 14.03.1991
(51) Int. Cl.: C07K 7/23

(54) **TREATMENT OF POLYCYSTIC OVARIAN DISEASE**
BEHANDLUNG POLYZYSTISCHER OVARKRANKHEIT
TRAITEMENT DE LA POLYKYSTOSE OVARIENNE

(30) Priority: 16.03.1990 GB 9005958; 25.05.1990 GB 9011823
(43) Date of publication of application: 04.03.1992
(73) Proprietor: APPLIED RESEARCH SYSTEMS ARS HOLDING N.V., Curacao (AN)
(72) Inventor: LUNENFELD, Bruno, 61 999 Tel Aviv (IL)
(74) Representative: Jones, Helen Marjorie Meredith
(86) International application number: EP9100479
(87) International publication number: WO9114704

(56) References cited:
- WO-A-90/14839
- CHEMICAL ABSTRACTS, vol. 86, no. 7, 14 February 1977, Columbus, OH (US); R.W. SHAW, p. 59, AN 37939q

## Description

The present invention relates to the treatment of polycystic ovarian disease (PCOD), and in particular a treatment for infertility associated therewith.

PCOD is a complex syndrome comprising a disorder of multiple etiologies involving a vicious circle of imbalance between various interdependent endocrine and peripheral structures. The syndrome is characterised by a variety of symptoms. Some or all of which may be present. These include menstrual abnormalities, hyperandrogenism, infertility and bilateral polycystic ovaries. Observations on the levels as well as the secretion and matabolism of the sex hormones helps to identify the pathophysiology of the syndrome.

Recently, several reports appeared showing that polycystic ovary disease may be connected with acanthosis nigricans and insulin resistance. (For instance see: Kahn CR, Flier JS, Bar RS, Archer JA, Gordon P, Martin MM, Roth J: The syndrome of insulin resistance and acanthosis nigricans. Insulin-receptor disorders in man. New Engl J Med 294:739, 1976; Burghen GA, Givens JR, Kitabachi AE: Correlation of hyperandrogenism with hyperinsulinism in polycystic ovarian disease. J Clin Endocrinol Metab 50:113, 1980; and Shapiro AG: (1981). Pituitary adenoma, menstrual disturbance, hirsutism and abnormal glucose tolerance. Fertil Steril 35: 226, 1981.) This indicates clearly that PCOD may be linked with insulin action and its control.

It also became apparent that growth factors (GF) play a modulating role in the ovarian response to gonadotropic stimulation as described by Adashi EA, Resnick CE, Svoboda ME, van Wyk JJ: Somatomedin C enhances induction of LH receptors by FSH in cultured rat granulosa cells. Endocrinol 116:2369, 1988. Homburg et al (Growth hormone facilitates ovulation induction by gonadotropins. Clin Endocrinol 29:113, 1988) demonstrated that the addition of growth hormone (hGH) to hMG therapy reduced the amount of gonadotropins required for ovulation induction. Blumenfeld & Lunenfeld (The potentiating effect of growth hormone on follicle stimulation with numan menopausal gonadotropins in a panhypopituitary patient. Fertil Steril 25:238, 1989.) demonstrated that patients with panhypopituitarism require excessive amounts of gonadotropins which can be reduced by concomitant administration of growth hormone. Menashe et al (Does endogenous hormone reserve correlate to ovarian response to menopausal gonadotropins? Isr J Med Sci 25:296, 1889) showed that anovulatory woman with reduced growth hormone reserve (as established by the clonidine growth hormone reserve test) needed significantly more gonadotropins to induce follicular maturation and ovulation than women who were clonidine positive.

Urdl, in polycystic ovarian disease: Endocrinological parameters with specific reference to growth hormone and somatomedin-C. Arch Gynecol Obstet 243:13, 1988, studied 33 women with polycystic ovarian disease and in 18 of them observed decreased hGH levels and increased Somatomedin-C (Sm-C) values. Pekonen et al in Decreased 34K insulin-like growth factor binding protein in polycystic ovarian disease. Fertil Steril 51:972, 1989, found that patients with PCOD had a decreased levels of human insulin-like growth factor-1 binding protein (hIGFBP-1).

All these observations indicate definitely that growth hormone and other growth factors as well as their binding proteins may play an important role in pathophysiology of PCOD.

Growth hormone stimulates the systemic release of insulin-like growth factor (IGF-1) from the liver. GH and probably other growth factors binding protein is also produced by the liver. Moreover, Leung and co-workers in Growth hormone receptor and serum binding protein: purification, cloning and expression. Nature 330:537, 1987, showed that the growth hormone receptor from rabbit liver and the growth hormone binding protein from rabbit serum have the same amino-terminal amino-acid sequence indicating that the binding protein corresponds to the extracellular hormone-binding domain of the liver receptor. It is becoming clear that the liver must play an important role in both normal and abnormal function of the ovaries.

AT this stage the inventors believe that PCOD is connected with higher levels of free IGF-1 (Somatomedin C). Since Somatomedin C increases the ovarian response to gonadotropins, this may explain the excessive production of andgrogens by the LH responsive structural ovarian components. Furthermore, it also explains the hyper-responsiveness of the ovarian follicular elements to FSH stimulation. If so, one pathophysiological basis of the PCOD could be explained as follows: the increased levels of free IGF-1 result in excessive follicular stimulation on the one hand and in overproduction of androgens leading to follicular atresia on the other hand.

PCOD has been treated by several schemes. Since the syndrome is associated with increased levels of androgen one treatment is to remove a section of androgen-producing tissue (ovarian wedge vesection) but his has now been replaced wherever possible by hormonal therapy. Administration of glucocorticoid reduces excessive androgen production mostly of adrenal origin and has been used with relative success in the management of PCOD treatment originating from adrenal disease. Antiestrogens such as clomiphene citrate have also been used.

Human menopausal gonadotropin (hMG) (eg a 50:50 mixture in I.U. of follicle stimulating hormone (FSH) and luteinising hormone (LH)) and FSH substantially free of LH have been used to treat infertile PCOD patients. FSH free of LH may be preferred as these patients are prone to hyperstimulation by LH. All gonadotropin therapy is subject to the risk of ovulation of multiple follicles and hyperstimulation. It has also been proposed to suppress endogenous secretion of gonadotropins by administration of a gonadotropin releasing hormone (GnRH) analogue prior to administration of exogenous gonadotropins and this does have some benefits as described by Coutts et al in Exerpta-Medica Int. Congress Series 652:608, 1984.

However since hIGFBP-1 and the level of free or bound IGF-1 are not affected by GnRH analogues, it is also logical that in this group of PCOD patients, the basic ovarian response to hMG or hFSH stimulation is not significantly changed by pituitary down regulation.

According to the present invention there is provided a new use of hIGFBP-1 protein increasing agents in the manufacture of a composition for use in a method of treatment of PCOD in which method said agent is administered in conjunction with a gonadotropin releasing hormone analogue.

In a further aspect of the invention there is provided the new use of a gonadotropin releasing hormone analogue in the manufacture of a composition for use in a method of treatment of PCOD in which method the gonadotropin releasing hormone analogue is administered in conjunction with an hIGFBP-1 increasing agent.

In the method the hIGFBP-1 increasing agent is preferably an estrogen.

The primary use of the method of treatment with which the present invention is concerned is in the treatment of infertility associated with PCOD. The method therefore usually includes induction of ovulation by using gonadotropins in the usual way. The induction of ovulation thus usually involves follicular maturation which is induced by the administration of human menopausal gonadotropins (eg a 50:50 mixture in I.U. of follicle stimulating hormone (FSH) and luteinising hormone (LH)) or of FSH substantially free of LH, followed by ovulation induction itself by human chorionic gonadotropin.

The administration of estrogen increases the level of IGF-1 binding globulin thus diminishing the excess of free IGF-1 available to the growing follicles. This has the consequence that the response of the ovaries to exogenous stimulation by gonadotropins will be proved and will be more reliable. Estrogen administration alone, in particular at high dose, would lead to an hormonal environment changing pituitary sensitivity so as to result in the release of excessive LH and this untimely release of LH would lead to anovulation. The administration of the GnRH analogues prevents the secretion of endogenous LH and FSH. Follicular development and ovulation are then induced in the normal way following the pituitary down regulation by GnRH analogue, by administration of exogenous hMG or FSH substantially free of LH and then hCG.

In the invention the GnRH analogue may be an agonist or an antagonist of GnRH. In general if it is an agonist then it is generally administered in a first cycle with estrogen and the gonadotropins adminstered in the following cycle. This allows the inhibitary action of the agonist to work. Where the analogue is an antagonist, then it may also be administered in a first cycle with the other components being administered in a succeeding cycle, but can also be administered co-jointly, that is over the same period as the other components.

Typical GnRH antagonists are described in Rees et al, J.Med. Cheml, 17, 1016 (1974), Coy et al, Peptides 1976 (Loffed Ed., Editions de L'Universite de Bruxelle 1977) p.463, Beattie et al, J.Med. Chem., 18, 1247 (1975), Channabasavaiah et al, Biochem. Biophys. Res. Commun., 86, 1266 (1979) and U.S. Patents 4,317,815 and 4,431,635, and include (Ac-pCl-Phe¹, pCl-Phe², D-Trp³, D-Arg⁶, D-Ala¹⁰)GnRH HCl, [D-Phe²]-LHRH, [D-Phe², D-Phe⁶]-LHRH, [D-Phe², Phe³, D-Phe⁶] -LHRH, [D-Phe², D-Trp³, D-Phe⁶]-LHRH, [D-p-F-Phe-D-Ala⁶]-LHRH, and [Ac-D-Phe¹, D-Phe², D-Trp^{3,6}]-LHRH.

The GnRH antagonist is administered in an amount which is sufficient to suppress endogeneous gonadotropin secretion. In general, the average daily dosage will be in the range of about 1.0-3.0 mg per kg and preferably in the range of about 1.5-2.5 mg/kg.

GnRH agonists are also known. One example is D-Ser (TBU) ⁶-EA¹⁰-LHRH (Hoe 766) and another is sold under the name Decapeptyl by CR.

In the invention the estrogen that is used is preferably an estradiol or a derivative thereof. A suitable derivative is estradiol benzoate. In general the estrogen is used in an effective amount for increasing IGF-1 binding globulin and thereby to decrease the amount of free IGF-1. The work of Urdl, cited above, suggests that the estrogen should be administered in relatively high doses.

The administration of estrogen and GnRH analogue create a hormonal and intrafollicular environment favourable for normal response to induction of ovulation with hMG or FSH substantially free of LH followed by hCG. The induction of ovulation is carried out in the manner described in EP-A-0161063, for instance the amount of gonadotropins used will generally be the same as in that reference.

The compositions in which the various active ingredients are supplied may be presented in the conventional forms, that is for oral, nasal or, preferably, parenteral administration, generally intramuscular administration. The various active ingredients may be provided in the same composition, where they can be administered at the same time, although usually are presented in separate compositions, which are thus suitable for co-joint use or for use over different periods.

The invention may be used for in vitro or in vivo fertilisation.

The following example outlines the regimen to be used for the method of treatment with which the present invention is concerned.

### Example

The following is a protocol by which the present invention will be assessed. In a method of treatment it is likely that some or all of the blood assays will not be carried out. The Clonodine test is likely to be carried out and will thus involve some or all of the blood assays. However, it is unlikely that it will be necessary to carry out the blood assays during the second cycle during administration of the estrodiol benzoate. Since the administration of FSH may be individually controlled and monitored the blood assays during that period may be carried out during the treatment itself.

The protocol is as follows:

### CYCLE 1

At the beginning of the preceding cycle a Clonidine test will be performed (2 Clonidine HCL tablets of 0,150 mg are administered orally.
Blood will be drawn and assayed for: FSH, LH, growth hormone (GH), estradiol (E-2), IGF-1, PRL at time 0 = before administration of Clonidine, and 30, 60, 90 and 120 min. following the administration of Clonidine GH will be measured. All these blood samples will be saved for future assays of IGF-1 and sex binding globulins. At the same day US scan of the ovaries will be performed.

GnRH analogue (Decapeptyl CR 3.2 mg) will be injected i.m on day 7th or 8th of the luteal phase of the cycle. Prior to the injection a beta-hCG test will be performed in order to exclude early pregnancy.

### CYCLE 2

On day 4 a US scan of the ovaries will be performed and blood will be drawn for: E-2, FSH, LH, GH, IGF-1 (possibly also for IGF-1 and sex binding globulins).

On day 4 1 mg of Estradiol Benzoate will be injected i.m.

On day 7 E-2, FSH, LH will be assayed.

On the same day 1 mg of Estradiol Benzoate will be injection i.m.

On day 10 E-2, FSH, LH, GH, IGF-1 (possibly also IGF-1 and sex binding globulins) will be assayed.

On the same day FSH (Metrodin, Teva-Serono) 150 IU will be administered i.m. and the treatment will be continued according to individually adjusted dose and monitored by daily E-2, FSH, LH assays and US scans.

If on day 16, ie after 6 days of Metrodin therapy no follicles greater than 17 mm and/or E-2 levels will not reach 350 pg/ml, Metrodin will be continued together with daily injections of Decapeptyl 0.1 mg i.m. until induction of ovulation will be possible. Ovulation will be induced by i.m. injection of 10,000 IU of hCG administered 24 h. after the final agonist dose. A US scan of the ovaries will be performed and blood will be taken for E-2, FSH, LH, GH IGF-1 (possibly also IFG-1 and sex binding globulins).

In cases subjected to IVF the above tests will be repeated on the day of ovum pick up and follicular fluid will be assayed for E-2, IGF-1 (possibly also IGF-1 and sex binding globulins).

## Claims (Claims for the following Contracting State(s): AT, BE, CH, LI, DE, DK, FR, GB, IT, LU, NL, SE)

1. Use of an hIGFBP-1 increasing agent in combination with a gonadotropin releasing hormone analogue in the manufacture of a pharmaceutical composition for the treatment of PCOD.

2. The use according to claim 1, wherein the hIGFBP-1 increasing agent and the GnRH analogue are administered simultaneously, separately or sequentially.

3. Use according to claim 1 or claim 2 in which the method includes the subsequent administration of gonadotropin(s) to induce ovulation.

4. Use according to claim 3 in which the gonadotropin administration comprises administration of hMG or of FSH substantially free of LH followed by the administration of hCG.

5. Use according to any preceding claim in which GnRH analogue is administered on a single day in a first cycle and estrogen is administered in the succeeding cycle.

6. Use according to claim 3 in which the said agent is administered on days 4 and 7 of the second cycle.

7. Use according to claim 4 and 6 in which the hMG or FSH is administered on day 10 of the second cycle and daily thereafter until ovulation is induced by hCG.

8. Use according to any preceding claim in which the hIGFBP-1 increasing agent is an estrogen.

9. Use according to claim 8 in which the estrogen is estradiol or a derivative.

10. Use according to any preceding claim in which GnRH analogue is a GnRH agonist.

11. A pharmaceutical composition containing an hIGFBP-1 increasing agent and a gonadotropin releasing hormone analogue in the presence of one or more pharmaceutically acceptable excipients for the simultaneous, separate or sequential administration of its active ingredients.

12. Product according to claim 11 in which the hIGFBP-1 increasing agent is an estrogen.

13. Product according to claim 11 or 12 containing also gonadotropins selected from human menopausal gonadotropins and FSH substantially free of LH.

## Claims (Claims for the following Contracting State(s): ES, GR)

1. Use of an hIGFBP-1 increasing agent in combination with a gonadotropin releasing hormone analogue in the manufacture of a pharmaceutical composition for the treatment of PCOD.

2. The use according to claim 1, wherein the hIGFBP-1 increasing agent and the GnRH analogue are administered simultaneously, separately or sequentially.

3. Use according to claim 1 or claim 2 in which the method includes the subsequent administration of gonadotropin(s) to induce ovulation.

4. Use according to claim 3 in which the gonadotropin administration comprises administration of hMG or of FSH substantially free of LH followed by the administration of hCG.

5. Use according to any preceding claim in which GnRH analogue is administered on a single day in a first cycle and estrogen is administered in the succeeding cycle.

6. Use according to claim 3 in which the said agent is administered on days 4 and 7 of the second cycle.

7. Use according to claim 4 and 6 in which the hMG or FSH is administered on day 10 of the second cycle and daily thereafter until ovulation is induced by hCG.

8. Use according to any preceding claim in which the hIGFBP-1 increasing agent is an estrogen.

9. Use according to claim 8 in which the estrogen is estradiol or a derivative.

10. Use according to any preceding claim in which GnRH analogue is a GnRH agonist.

11. Process for the manufacture of a pharmaceutical composition for the treatment of PCOD which comprises incorporating an hIGFBP-1 increasing agent, a gonadotropin releasing hormone (GnRH) analogue and one or more pharmaceutically acceptable excipients into a composition wherein the active ingredients are arranged for simultaneous, separate or sequential administration.

12. Process according to claim 11 in which the hIGFBP-1 increasing agent is an estrogen.

13. Process according to claim 12 in which the estrogen is estradiol or a derivative.

14. Process according to any of claims 11 to 13 in which the GnRH analogue is a GnRH agonist.

15. Process according to any of claims 11 to 14 in which gonadotropins selected from human menopausal gonadotropins and FSH substantially free of LH is incorporated into the composition.

## Patentansprüche (Patentansprüche für folgende(n) Vertragsstaat(en): AT, BE, CH, LI, DE, DK, FR, GB, IT, LU, NL, SE)

1. Verwendung eines hIGFBP-1-erhöhenden Agens in Kombination mit einem Gonadotropin-freisetzendes-Hormon-Analogon zur Herstellung einer pharmazeutischen Zusammensetzung zur Behandlung von PCOD.

2. Verwendung nach Anspruch 1, worin das hIGFBP-1-erhöhende Agens und das GnRH-Analogon gleichzeitig, separat oder nacheinander verabreicht werden.

3. Verwendung nach Anspruch 1 oder Anspruch 2, worin das Verfahren die anschließende Verabreichung von Gonadotropin(en) zur Induktion der Ovulation umfaßt.

4. Verwendung nach Anspruch 3, worin die Gonadotropin-Verabreichung umfaßt die Verabreichung von hMG oder von FSH, im wesentlichen frei von LH, gefolgt von der Verabreichung von hCG.

5. Verwendung nach irgendeinem der vorhergehenden Ansprüche, worin das GnRH-Analogon an einem einzigen Tag in einem ersten Zyklus verabreicht wird und Estrogen im nachfolgenden Zyklus verabreicht wird.

6. Verwendung nach Anspruch 3, worin das Agens an den Tagen 4 und 7 des zweiten Zyklus verabreicht wird.

7. Verwendung nach Anspruch 4 und 6, worin das hMG oder FSH am Tag 10 des zweiten Zyklus verabreicht wird und danach täglich, bis die Ovulation durch hCG induziert wird.

8. Verwendung nach irgendeinem der vorhergehenden Ansprüche, worin das hIGFBP-1-erhöhende Agens ein Estrogen ist.

9. Verwendung nach Anspruch 8, worin das Estrogen Estradiol oder ein Derivat ist.

10. Verwendung nach irgendeinem der vorhergehenden Ansprüche, worin das GnRH-Analogon ein GnRH-Agonist ist.

11. Pharmazeutische Zusammensetzung, enthaltend ein hIGFBP-1-erhöhendes Agens und ein Gonadotropin-freisetzendes-Hormon-Analogon in Gegenwart von einem oder mehreren pharmazeutisch annehmbaren Excipienten, für die gleichzeitige, separate oder aufeinanderfolgende Verabreichung ihrer wirksamen Bestandteile.

12. Produkt nach Anspruch 11, worin das hIGFBP-1-erhöhende Agens ein Estrogen ist.

13. Produkt nach Anspruch 11 oder 12, enthaltend auch Gonadotropine, die aus Human-Menopausen-Gonadotropinen und FSH, im wesentlichen frei von LH, ausgewählt sind.

## Patentansprüche (Patentansprüche für folgende(n) Vertragsstaat(en): ES, GR)

1. Verwendung eines hIGFBP-1-erhöhenden Agens in Kombination mit einem Gonadotropin-freisetzendes-Hormon-Analogon zur Herstellung einer pharmazeutischen Zusammensetzung zur Behandlung von PCOD.

2. Verwendung nach Anspruch 1, worin das hIGFBP-1-erhöhende Agens und das GnRH-Analogon gleichzeitig, separat oder nacheinander verabreicht werden.

3. Verwendung nach Anspruch 1 oder Anspruch 2, worin das Verfahren die anschließende Verabreichung von Gonadotropin(en) zur Induktion der Ovulation umfaßt.

4. Verwendung nach Anspruch 3, worin die Gonadotropin-Verabreichung umfaßt die Verabreichung von hMG oder von FSH, im wesentlichen frei von LH, gefolgt von der Verabreichung von hCG.

5. Verwendung nach irgendeinem der vorhergehenden Ansprüche, worin das GnRH-Analogon an einem einzigen Tag in einem ersten Zyklus verabreicht wird und Estrogen im nachfolgenden Zyklus verabreicht wird.

6. Verwendung nach Anspruch 3, worin das Agens an den Tagen 4 und 7 des zweiten Zyklus verabreicht wird.

7. Verwendung nach Anspruch 4 und 6, worin das hMG oder FSH am Tag 10 des zweiten Zyklus verabreicht wird und danach täglich, bis die Ovulation durch hCG induziert wird.

8. Verwendung nach irgendeinem der vorhergehenden Ansprüche, worin das hIGFBP-1-erhöhende Agens ein Estrogen ist.

9. Verwendung nach Anspruch 8, worin das Estrogen Estradiol oder ein Derivat ist.

10. Verwendung nach irgendeinem der vorhergehenden Ansprüche, worin das GnRH-Analogon ein GnRH-Agonist ist.

11. Verfahren zur Herstellung einer pharmazeutischen Zusammensetzung zur Behandlung von PCOD, welches umfaßt die Inkorporierung eines hIGFB-1 erhöhenden Agens, eines Gonadotropin-freisetzendes-Hormon (GnRH) -Analogons und eines oder mehrerer pharmazeutisch annehmbarer(n) Excipienten in eine Zusammensetzung, worin die wirksamen Bestandteile zur gleichzeitigen, separaten oder aufeinanderfolgenden Verabreichung angeordnet sind.

12. Verfahren nach Anspruch 11, worin das hIGFBP-1-erhöhende Agens ein Estrogen ist.

13. Verfahren nach Anspruch 12, worin das Estrogen Estradiol oder ein Derivat ist.

14. Verfahren nach irgendeinem der Ansprüche 11 bis 13, worin das GnRH-Analogon ein GnRH-Agonist ist.

15. Verfahren nach irgendeinem der Ansprüche 11 bis 14, worin Gonadotropine, die aus Human-Menopausen-Gonadotropinen und FSH, im wesentlichen frei von LH, ausgewählt sind, in die Zusammensetzung inkorporiert werden.

## Revendications (Revendications pour l'(les) Etat(s) contractant(s) suivant(s): AT, BE, CH, LI, DE, DK, FR, GB, IT, LU, NL, SE)

1. Utilisation d'un agent augmentant hIGFBP-1 en association avec un analogue de la gonadolibérine dans la préparation d'une composition pharmaceutique destinée au traitement de la polykystose ovarienne.

2. Utilisation selon la revendication 1, dans laquelle l'agent augmentant hIGFBP-1 et l'analogue de GnRH sont administrés simultanément, séparément ou successivement.

3. Utilisation selon la revendication 1 ou la revendication 2, dans laquelle le procédé comprend l'administration subséquente de gonadotrophine(s) pour induire l'ovulation.

4. Utilisation selon la revendication 3, dans laquelle l'administration de gonadotrophine(s) comprend l'administration de hMG ou de FSH sensiblement exempte de LH, suivie de l'administration de hCG.

5. Utilisation selon l'une quelconque des revendications précédentes, dans laquelle l'analogue de GnRH est administré un seul jour au cours d'un premier cycle et l'oestrogène est administré au cours du cycle suivant.

6. Utilisation selon la revendication 3, dans laquelle ledit agent est administré le quatrième jour et le septième jour du deuxième cycle.

7. Utilisation selon la revendication 4 et 6, dans laquelle hMG ou FSH est administrée le dixième jour du deuxième cycle, puis quotidiennement jusqu'à ce que l'ovulation soit induite par hCG.

8. Utilisation selon l'une quelconque des revendications précédentes, dans laquelle l'agent augmentant hIGFBP-1 est un oestrogène.

9. Utilisation selon la revendication 8, dans laquelle l'oestrogène est l'estradiol ou un dérivé.

10. Utilisation selon l'une quelconque des revendications précédentes, dans laquelle l'analogue de GnRH est un agoniste de GnRH.

11. Composition pharmaceutique contenant un agent augmentant hIGFBP-1 et un analogue de la gonadolibérine en présence d'un ou plusieurs excipients pharmaceutiquement acceptables pour l'administration simultanée, séparée ou successive de ses principes actifs.

12. Produit selon la revendication 11, dans lequel l'agent augmentant hIGFBP-1 est un oestrogène.

13. Produit selon la revendication 11 ou 12, contenant également des gonadotrophines sélectionnées parmi des gonadotrophines humaines de femmes ménopausées et la FSH sensiblement exempte de LH.

## Revendications (Revendications pour l'(les) Etat(s) contractant(s) suivant(s): ES, GR)

1. Utilisation d'un agent augmentant hIGFBP-1 en association avec un analogue de la gonadolibérine dans la préparation d'une composition pharmaceutique destinée au traitement de la polykystose ovarienne.

2. Utilisation selon la revendication 1, dans laquelle l'agent augmentant hIGFBP-1 et l'analogue de GnRH sont administrés simultanément, séparément ou successivement.

3. Utilisation selon la revendication 1 ou la revendication 2, dans laquelle le procédé comprend l'administration subséquente de gonadotrophine(s) pour induire l'ovulation.

4. Utilisation selon la revendication 3, dans laquelle l'administration de gonadotrophine(s) comprend l'administration de hMG ou de FSH sensiblement exempte de LH, suivie de l'administration de hCG.

5. Utilisation selon l'une quelconque des revendications précédentes, dans laquelle l'analogue de GnRH est administré un seul jour au cours d'un premier cycle et l'oestrogène est administré au cours du cycle suivant.

6. Utilisation selon la revendication 3, dans laquelle ledit agent est administré le quatrième jour et le septième jour du deuxième cycle.

7. Utilisation selon la revendication 4 et 6, dans laquelle hMG ou FSH est administrée le dixième jour du deuxième cycle, puis quotidiennement jusqu'à ce que l'ovulation soit induite par hCG.

8. Utilisation selon l'une quelconque des revendications précédentes, dans laquelle l'agent augmentant hIGFBP-1 est un oestrogène.

9. Utilisation selon la revendication 8, dans laquelle l'oestrogène est l'estradiol ou un dérivé.

10. Utilisation selon l'une quelconque des revendications précédentes, dans laquelle l'analogue de GnRH est un agoniste de GnRH.

11. Procédé de préparation d'une composition pharmaceutique destinée au traitement de la polykystose ovarienne, lequel comprend l'incorporation d'un agent augmentant hIGFBP-1, d'un analogue de la gonadolibérine (GnRH) et d'un ou plusieurs excipients pharmaceutiquement acceptables dans une composition où les principes actifs sont disposés pour l'administration simultanée, séparée ou successive.

12. Procédé selon la revendication 11, dans lequel l'agent augmentant hIGFBP-1 est un oestrogène.

13. Procédé selon la revendication 12, dans lequel l'oestrogène est l'estradiol ou un dérivé.

14. Procédé selon l'une quelconque des revendications 11 à 13, dans lequel l'analogue de GnRH est un agoniste de GnRH.

15. Procédé selon l'une quelconque des revendications 11 à 14, dans lequel des gonadotrophines sélectionnées parmi des gonadotrophines humaines de femmes ménopausées et la FSH sensiblement exemple de LH sont incorporées dans la composition.
